Europäisches Patentamt

European Patent Office

Office européen des brevets

(11) Veröffentlichungsnummer: **0 195 967**
**A2**

## EUROPÄISCHE PATENTANMELDUNG

(21) Anmeldenummer: **86102987.4**

(22) Anmeldetag: **06.03.86**

(51) Int. Cl.⁴: **A 01 M 13/00,** A 01 M 1/20,
A 61 L 9/03

(30) Priorität: **23.03.85 DE 3510641**

(43) Veröffentlichungstag der Anmeldung: **01.10.86**
**Patentblatt 86/40**

(84) Benannte Vertragsstaaten: **DE FR GB IT**

(71) Anmelder: **Napierski, Reinhard, Talstrasse 18,**
**D-6361 Niddatal 1 (DE)**

(72) Erfinder: **Napierski, Reinhard, Talstrasse 18,**
**D-6361 Niddatal 1 (DE)**

(74) Vertreter: **Jochem, Bernd, Dipl.-Wirtsch.-Ing.,**
**Patentanwälte Beyer & Jochem Postfach 17 01 45,**
**D-6000 Frankfurt/Main (DE)**

(54) **Elektrisches Gerät zum Verdampfen von in Trägerplättchen enthaltenen Wirkstoffen.**

(57) Es wird ein Gerät zum Verdampfen von in Trägerplättchen enthaltenen Wirkstoffen beschrieben. Dabei werden Heizung (12), Zuleitung (14) und Kontaktstifte (16) in zwei Halbschalen eingeschlossen. Die Halbschalen bilden gleichzeitig das Schutzgitter (18) und Niederhalter (38), die das Trägerplättchen an den Heizblock (22) herandrücken. Hierbei dienen Leisten als seitliche Führung. Der Heizblock (22) steht relativ frei im Inneren und ist nur über Füße (15) nach außen verbunden. Wandungen um den Heizblock (22) verhindern den direkten Zugriff zum Heizblock (22).

Die Erfindung bezieht sich auf ein Gerät zum Verdampfen von in Trägerplättchen enthaltenen Wirkstoffen, insbesondere Insektiziden und Desodorants, bestehend aus einem Gehäuse, welches eine Heizung aufnimmt, wobei einzelne Trägerplättchen an einer Heizfläche zur Anlage kommen.

Ein derartiges Gerät ist in der DE-PS 27 30 855 beschrieben. Es hat ein fest mit dem Gehäuse verbundenes Schutzgitter, unter dem sich eine an beiden Enden offene Durchschiebeführung erstreckt. Kufenartige Niederhalter auf der Innenseite des Schutzgitters im Bereich der Durchschiebeführung sorgen dafür, daß ein in die Durchschiebeführung eingeführtes Wirkstoffplättchen an die Heizplatte angedrückt wird. Dort besteht der Heizblock aus einem Keramikteil mit einzementiertem Heizwiderstand und losen Zuleitungen zu den Kontaktstiften. Eine Verbesserung der Montage des Verdampfers wurde in der DE-AS 28 32 249 beschrieben. Dort wurde der Heizblock mit Kontaktstiften versehen, die in Steckkontakt-Buchsen der Kontaktstifte zur schnelleren Montage und Austauschbarkeit lösbar eingesteckt werden.

Diese bekannten Ausführungen ermöglichen nicht das freie Stehen des Heizblocks im Gehäuse. Abgesehen von den Zuleitungen zum Heizblock muß der Heizblock am Gehäuse nochmals zusätzlich abgestützt bzw. in Position gehalten werden. Dieses hat den Nachteil, daß an dieser Übergangsstelle die Wärme vom Heizblock zum Gehäuse übertragen wird. Die vorgeschlagenen Geräte bestehen zudem aus vielen Einzelteilen, die separat gefertigt und montiert werden müssen.

Der Erfindung liegt deshalb die Aufgabe zugrunde,
ein Gerät der eingangs genannten Art zu schaffen,
für das ein Minimum an Teilen benötigt wird, die
sich leicht und schnell komplettieren lassen.

Vorstehende Aufgabe wird erfindungsgemäß durch die
Gesamtheit der Merkmale des Anspruchs 1 gelöst.
Dabei beruht der Vorteil der Einfachheit vor allem
auf dem Gedanken, daß der Heizwiderstand von dem
gleichen Kunststoff umschlossen wird, aus dem auch
das Gehäuse besteht.

Um eine sehr einfache Ausführung zu erhalten, ist
in bevorzugter Ausführung der Erfindung vorgesehen,
daß zwei gleiche Kunststoffhalbschalen die Heizung,
elektrische Zuleitung und Kontaktstifte aufnehmen
und gleichzeitig eine Durchschiebeöffnung für die
Trägerplättchen bilden. Die Kunststoffhalbschalen
bilden auch gleichzeitig um den Heizblock Wände,
die den Zugriff von außen zum heißen Heizblock verhindern.

Somit ist es nur erforderlich, die Heizung, Zuleitung
und Kontaktstifte miteinander zu verbinden und diese
Einheit in eine Halbschale einzulegen, um dann mit
der anderen Halbschale zum kompletten Gerät zu verschließen. Die beiden Halbschalen können miteinander
verschweißt oder unlösbar verhakt werden. Es besteht
durchaus auch die Möglichkeit, eine kleinere Einheit
aus den zwei Halbschalen herzustellen, z.B. könnte
man nur die Heizung, Zuleitung und Kontaktstifte mit
den beiden Halbschalen umschließen und dann diese
Einheit in ein anderes bereits bekanntes Gehäuse einbauen. Dieses hätte den Vorteil, daß die Halbschalen,
die die Heizung umschließen, aus einem wärmefesten
Kunststoff hergestellt werden könnten.

Der zusätzliche Vorteil dieser Konstruktion ist,
daß Heizungen montiert werden können, deren Außentemperatur über der Schmelztemperatur des verwendeten Kunststoffes liegen kann.

Eine weitere Problemlösung und Ausgestaltung der
Erfindung ermöglicht, daß die elektrische Zuleitung
von außen zur innenliegenden elektrischen Heizung
eine unlösbare Einheit bildet und von einem einstückigen elektrisch isolierenden Material umgeben
ist, das im wesentlichen und unmittelbar die elektrische Zuleitung starr umschließt.

Die Erfindung ermöglicht hierdurch eine einfache
Herstellung eines Verdampfers, in dem die Widerstandsheizung mit den elektrischen Anschlüssen in
einem Spritz- oder Preßverfahren von einer isolierenden Masse umschlossen wird, die gleichzeitig die
Heizfläche bildet. Es spielt hierbei keine Rolle,
ob die Heizung in einem Keramikkörper eingebettet
ist. In einfacher Ausführung ist es nur erforderlich, die Widerstandsspirale dünnwandig zu beschichten.

In bevorzugter Ausgestaltung der Erfindung besteht
der Verdampfer einstückig aus einem elektrisch isolierenden Material, z.B. wärmefestem Kunststoff,
das die Widerstandsheizung und teilweise die elektrischen Kontaktstifte unmittelbar umschließt und
gleichzeitig ein Schutzgitter bildet, so daß z.B. in
einer Spritzform ein Heizwiderstand verbunden mit
den elektrischen Kontaktstiften hereingelegt wird
und abgespritzt wird. Somit wird durch einen einzigen Spritzvorgang ein Verdampfer komplettiert.

Die bevorzugte Herstellung im Spritzgußverfahren ist mit den zur Zeit in den bekannten Verdampfern eingesetzten 5-W-Heizwiderständen schlecht möglich, da diese zu hohe Außentemperaturen bei einer Oberfläche von 230 mm$^2$ haben. Es ist deshalb zweckmäßig, den Heizwiderstand anzupassen, so daß bei gleicher Leistung, ca. 5 W, die Oberfläche des Heizwiderstandes auf über 450 mm$^2$ ausgelegt wird. Hierbei ist es nicht nötig, den Heizwiderstand in ein Keramikgehäuse einzuzementieren, sondern die Heizspirale wird nur auf einen dickeren Träger aufgewickelt und festgelegt.

Eine weitere zweckmäßige Ausgestaltung der Erfindung ist, daß das Elektroteil - bestehend aus den eingeschlossenen Teilen: Widerstandsheizung und elektrischen Kontaktstiften - und das Schutzgitter zwei voneinander getrennte Teile sind, die zum Schluß miteinander montiert werden. Diese Ausführung ermöglicht, einen Verdampfer herzustellen, der für hohe Temperaturen ausgelegt ist, denn auf diese Weise kann das Elektroteil aus einem wärmefesteren Kunststoff oder Keramik bestehen und das Schutzgitter aus einem einfacheren und billigen Kunststoff hergestellt werden.

Um das Elektroteil, bestehend aus Heizblock und Steckerteil, in einem Spritzgußverfahren abzuspritzen, müssen die eingelegten Teile, wie Widerstandsheizung, Zuleitung und Kontaktstifte, in die Spritzgußform eingelegt und arretiert werden. Deshalb werden bevorzugt an diesen Arretierstellen, abgesehen von den Kontaktstiften, isolierte Stellen vorgesehen, damit das dann abgespritzte Teil voll isoliert ist.

Es besteht auch die Möglichkeit, die Zuleitung von den Kontaktstiften zur Widerstandsheizung starr oder einstückig mit den Kontaktstiften auszulegen, so daß nur eine Arretierung an den Kontaktstiften nötig ist.

Die Erfindung wird nachstehend anhand von in der Zeichnung dargestellten Ausführungsbeispielen näher erläutert. Es zeigen:

Fig. 1     eine offene Halbschale gemäß der
           Erfindung;

Fig. 2     einen Querschnitt durch das Gerät
           gemäß Fig. 1;

Fig. 3     ein weiteres Ausführungsbeispiel in
           einer Ansicht  entsprechend Fig. 1;

Fig. 4     einen Querschnitt durch das Gerät
           gemäß Fig. 3;

Fig. 5 bis 10   weitere Ausführungsbeispiele
                gemäß der Erfindung.

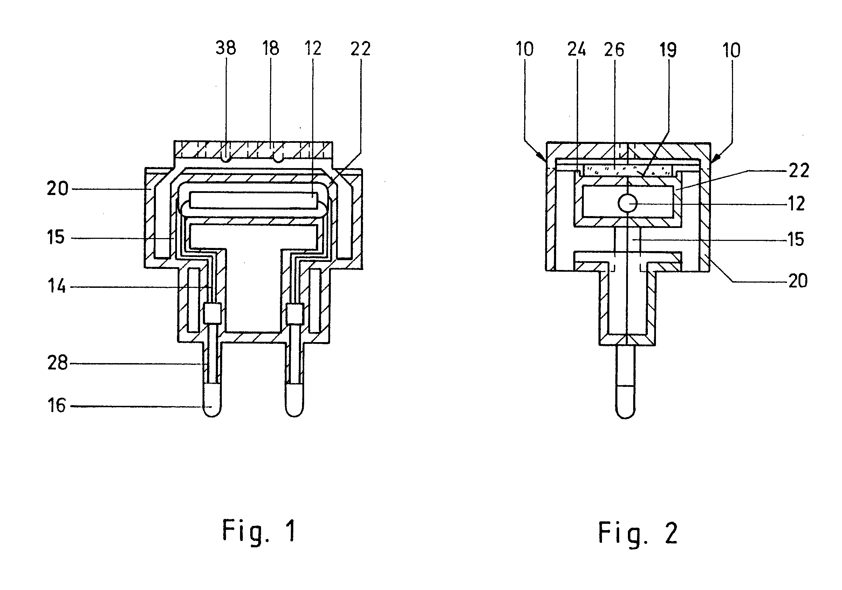

Das Gerät in Fig. 1 und Fig. 2 besteht im Beispielsfalle aus zwei Halbschalen 10, in denen die elektrische Heizung 12, Zuleitung 14 und teilweise Kontaktstifte 16 eingeschlossen sind. Die Halbschalen 10 bilden gleichzeitig das Schutzgitter 18 und die Wandungen 20, um den direkten Zugriff von außen zum Heizblock 22 zu verhindern.

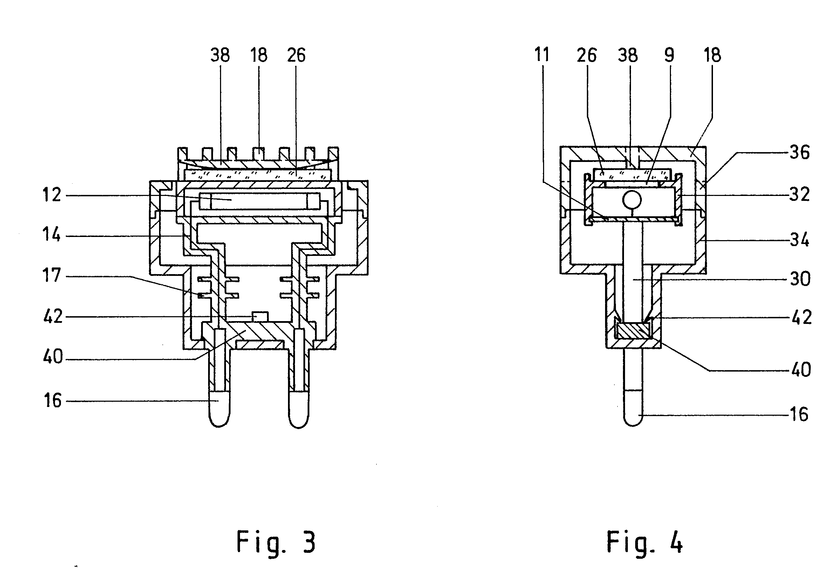

Die Halbschalen 10 bilden auch den Heizblock 22, in dem die Heizung 12 nach allen Seiten frei angeordnet ist, damit die relativ hohe Temperatur der Heizung 12 nicht die Wandung des Heizblocks 22 anschmilzt. Der Heizblock 22 ist auch nach allen
Seiten frei im Gehäuse gehalten. Die einzige Verbindung zwischen Heizblock 22 und Außenwandung
besteht nur über schmale Füße 15, die gegebenerweise die Zuleitung 14 aufnehmen. Diese Füße 15
sind der einzige direkte Wärmeübergang nach außen
und können je nach Temperatur des Heizblocks 22
zusätzlich mit Kühlrippen 17, wie in Fig. 3 gezeigt, ausgelegt werden.

Diese Konstruktion ermöglicht die Abgrenzung von
Temperaturzonen, und zwar die Heizung 12 vom Heizblock 22 und den Heizblock 22 vom Außengehäuse.

Am Heizblock 22 sind Führungsleisten 24 angeordnet,
um das Trägerplättchen 26 auf dem Heizblock 22 zu
führen. Um die Kontaktstifte 16 in der gewünschten
Position zu halten, sind Verjüngungen 28 der Kontaktstifte 16 vorgesehen, die im Bereich der Halbschalen 10 liegen. Zur besseren Wärmeübertragung
an das Trägerplättchen 26 und Belüftung des Heizblocks 22 sind Aussparungen 9 vorgesehen.

Die beiden Halbschalen 10 können identisch ausgelegt werden und somit aus einem Spritzwerkzeug gefertigt werden.
Fig. 1 zeigt eine offene Halbschale 10 (Berührungsflächen der Halbschalen schraffiert gezeichnet), in
der die Heizung 12, Zuleitung 14 und Kontaktstifte
16 vormontiert hereingelegt sind. Es ist jetzt nur
noch nötig, die zweite Halbschale 10 draufzulegen
und die beiden Halbschalen 10 dann unlösbar, z.B.
durch Schweißen oder sonstige Verzahnung, zu verbinden.

Das Gerät in Fig. 3 und Fig. 4 besteht aus einem
einstückig gespritzten oder gepreßten Elektroteil 30,
enthaltend . Heizung 12, Zuleitung 14, Kontaktstifte 16 mit Platte 11, das mit einer U-förmigen
Abdeckung 32 eine komplette Einheit bildet, die in
zwei Schalen 34 und 36 beliebiger Kontur montiert
werden können. Die Schale 36 bildet das Schutzgitter 18 und gleichzeitig den Niederhalter 38, der das
Trägerplättchen 26 auf die Abdeckung 32 drückt.
Auch hierbei ist der gebildete Heizblock frei in den
umgebenden Schalen 34 und 36; es besteht nur eine
Verbindung bei der Zuleitung 14, die über eine
Brücke 40 und Nasen 42 in der Schale 34 gehalten
wird.

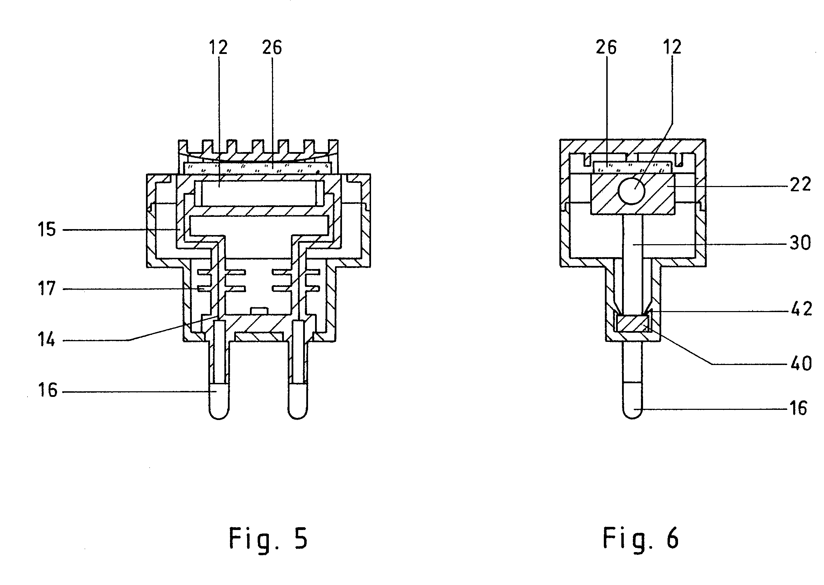

Das Ausführungsbeispiel nach Fig. 5 und 6 entspricht
der Ausführung nach Fig. 3 und 4, nur mit dem Unterschied, daß das Elektroteil 30 in einem Spritzvorgang einstückig die Heizung 12, Zuleitung 14 und
Kontaktstifte 16 umschließt und somit eine komplette
Einheit bildet.

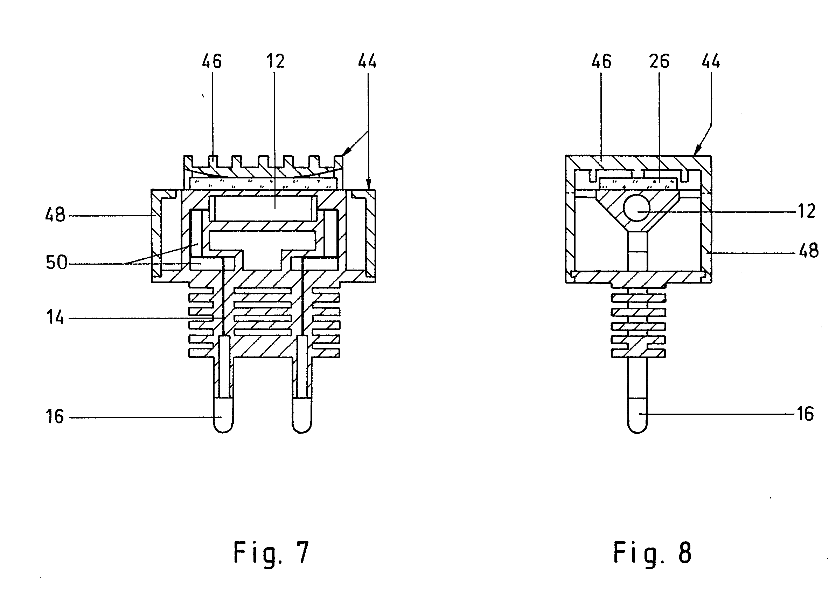

In Fig. 7 und 8 wird eine weitere Variante gezeigt,
bei der die eingeschlossenen Elektroteile 12, 14, 16
und die untere Schale einstückig ausgebildet sind,
so daß es nur nötig ist, die obere Schale 44 zu montieren. Die obere Schale 44 beinhaltet das Schutzgitter 46 und die äußeren Wandungen 48.

Um Einschlüsse 50 zu vermeiden, können die Zuleitung 14 und die Kontaktstifte 16 einstückig und
starr ausgelegt werden. Bei labiler Zuleitung 14
müssen beim Einlegen in ein Spritzwerkzeug die elektrischen Einheiten 12, 14 und 16 arretiert werden, und
dies erfolgt an den Einschlüssen 50. Diese Einschlüsse 50 können natürlich vorher isoliert werden, damit dann aus dem Spritzwerkzeug ein komplett isolierter Körper erstellt wird. Dieses ist jedoch zu vernachlässigen, wenn die Einschlüsse 50 durch ein zusätzlich montiertes Teil 48 sowieso abgedeckt werden.

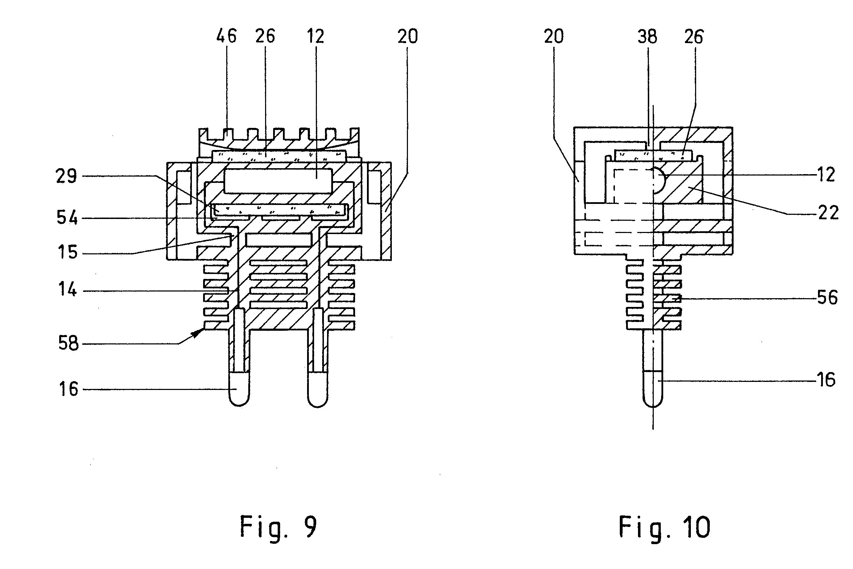

Fig. 9 und 10 zeigt eine Ausführung eines Verdampfers, die in einem Spritzvorgang oder Preßvorgang hergestellt werden kann. Die Elektroteile 12, 14 und 16 werden in ein Spritzwerkzeug hineingelegt und abgespritzt, somit kann man durch nur einen Spritzvorgang einen kompletten Verdampfer herstellen, der das Grundmerkmal des relativ freien Heizblocks 22 beinhaltet.

Auch in dieses Gerät wird ein Wirkstoffplättchen 26 zwischen Heizblock 22 und Schutzgitter 18 eingeschoben und mittels Niederhalter 38 auf der Heizfläche gehalten. Es versteht sich, daß die Zuführung der Wirkstoffplättchen 26 auch eine andere sein kann als beschrieben, z.B. mit Zuhilfenahme eines zusätzlichen Teils in Form eines Rahmens, können die Wirkstoffplättchen 26 von außen nach innen auf die Heizfläche transportiert und umgekehrt auch wieder entnommen werden.

Die Konstruktion erlaubt auch, daß ein zusätzliches Wirkstoffplättchen 29 unter dem Heizblock 22 (zwischen Heizblock 22 und Steckerteil 58) zum Verdampfer eingeschoben werden kann. Der Vorteil ist, daß doppelt so große Räume mittels nur eines Geräts geschützt werden können; oder man könnte auch ein Insektizid- und Deo-Wirkstoffplättchen gleichzeitig verdampfen. Da die Insektizid- und Deo-Wirkstoffe unterschiedliche Temperaturen zum Verdampfen benötigen, wird die Heizung 12 einseitig im Heizblock 22

0195967

eingeschlossen, so daß Temperaturunterschiede am Heizblock 22 entstehen, oder das Deo-Wirkstoffplättchen wird mit einem gewissen Abstand vom Heizblock 22 eingeschoben. Zwischen dem zweiten Einschiebekanal 54 und dem Steckerteil 58 sind Kühlrippen 56 vorgesehen, die verhindern, daß das Steckerteil 58 aufgeheizt wird.

Anstelle der Kontaktstifte können auch Kabelausgänge, die zu einem elektrischen Stecker führen, als Einheit ausgelegt sein.

Die Konstruktion erlaubt, auch jede andere Außenform als die gezeigte zu gestalten, denn hierzu benötigt man nur eine weitere Anformung von Rippen oder ganze gerippte Körperteile. Die Schutzgitter 18, 46 mit den Schutzrippen können als Hohlkörper beliebig geformt werden.

Um eine bessere Wärmeleitung von der Heizung 12 über das isolierende Material zu bekommen, kann im umspritzten oder umpreßten Kunststoff keramisches Granulat eingelagert werden.

## Patentansprüche

1. Elektrisches Gerät zum Verdampfen von in Träger-plättchen enthaltenen Wirkstoffen, insbesondere Insektiziden und Desodorants, bestehend aus einem Gehäuse, welches eine Heizung aufnimmt, wobei einzelne Trägerplättchen an einer Heizfläche zur Anlage kommen, d a d u r c h g e k e n n - z e i c h n e t, daß im wesentlichen frei im Gehäuse (10) ein Heizblock (22) aus isolierendem Material mit herausgeführten elektrischen Zuleitungen (14) angeordnet ist, in dem im wesentlichen die elektrische Heizung (12) umschlossen ist.

2. Gerät nach Anspruch 1, d a d u r c h g e - k e n n z e i c h n e t, daß der Heizblock (22) gebildet wird aus mindestens zwei Kunststoffteilen, die die Heizung (12) umschließen.

3. Gerät nach Anspruch 2, d a d u r c h g e - k e n n z e i c h n e t, daß der Heizblock (22) aus mindestens zwei gleichen Kunststoffschalen gebildet wird.

4. Gerät nach Anspruch 1, d a d u r c h g e - k e n n z e i c h n e t, daß zwei gleiche Kunst-stoffschalen die elektrische Heizung (12), Zuleitung (14) und Kontaktstifte (16) umschließen.

5. Gerät nach Anspruch 4, d a d u r c h g e - k e n n z e i c h n e t, daß die zwei Kunst-stoffschalen (10) die Heizung (12), Zuleitung (14) und Kontaktstifte (16) umschließen und gleichzeitig eine Abschirmung der Heizung (12) bilden.

6. Gerät nach Anspruch 4, d a d u r c h   g e -
k e n n z e i c h n e t , daß die zwei Kunst-
stoffschalen (10) die elektrischen Teile umschließen und gleichzeitig das Schutzgitter (18)
und Wandung (20) bilden.

7. Gerät nach Anspruch 1, d a d u r c h   g e -
k e n n z e i c h n e t , daß eine Kunststoffschale (10) die Heizung (12), Zuleitung (14) und
Kontaktstifte (16) aufnimmt und diese arretiert.

8. Gerät nach Anspruch 1, d a d u r c h   g e -
k e n n z e i c h n e t , daß der Heizblock (22)
im Gehäuse bis auf zwei Füße (15) berührungsfrei
angeordnet ist.

9. Gerät nach Anspruch 1, d a d u r c h   g e -
k e n n z e i c h n e t , daß die Heizung (12),
Zuleitung (14) und Kontaktstifte (16) eine Einheit bilden, wobei die Zuleitung (14) und Kontaktstifte (16) einstückig mit einem elektrisch
isolierenden Material unmittelbar umschlossen
sind.

1o. Gerät nach Anspruch 1, d a d u r c h   g e -
k e n n z e i c h n e t , daß die elektrische
Heizung (12) im Heizblock (22) berührungsfrei
angeordnet ist und nur an den Zuleitungen (14)
gehalten wird.

11. Gerät, insbesondere nach einem der Ansprüche 1 - 1o, dadurch gekennzeichnet, daß die elektrische Zuleitung (14) von außen zur innenliegenden elektrischen Heizung (12) mit dieser eine unlösbare Einheit bildet, die umgeben ist von einem einstückigen elektrisch isolierenden Material, das sie im wesentlichen und unmittelbar umschließt.

Fig. 2

Fig. 1

Fig. 4

Fig. 3

Fig. 5

Fig. 6

Fig. 8

Fig. 7

Fig. 9

Fig. 10

0195967